# EUROPEAN PATENT SPECIFICATION

(11) **EP 4 583 080 B1**
(45) Date of publication and mention of the grant of the patent: **04.11.2026**
(21) Application number: 25163160.2
(22) Date of filing: 21.12.2018
(51) Int. Cl.: A61B 1/00, A61B 1/05, G02B 23/24, G09B 23/28

(54) **SURGICAL SIMULATION CAMERA SCOPE**
KAMERAENDOSKOP FÜR CHIRURGISCHE SIMULATION
LUNETTE DE CAMÉRA DE SIMULATION CHIRURGICALE

(30) Priority: 04.01.2018 US 201862613696 P; 30.08.2018 US 201862724720 P
(43) Date of publication of application: 09.07.2025
(62) Document number(s) of the earlier application(s) in accordance with Art. 76 EPC: 18836784.1 / 3 735 163
(73) Proprietor: Applied Medical Resources Corporation, Rancho Santa Margarita, CA 92688 (US)
(72) Inventor: DANTES, Richard, Rancho Santa Margarita, 92688 (US); FILEK, Jacob, Rancho Santa Margarita, 92688 (US); HO, Jimmy, Rancho Santa Margarita, 92688 (US); KENNEDAY, Sean, Rancho Santa Margarita, 92688 (US)
(74) Representative: Dolleymores

(56) References cited:
- DE-B3- 102004 023 866
- JP-A- 2002 291 693
- US-A- 4 491 865
- US-A- 5 325 847
- US-B2- 7 435 218

## Description

### Background

The present application relates to surgical simulation camera scopes.

Surgical scopes are used in endoscopic and laparoscopic procedures in which small incisions are employed to pass scopes and instruments, such as scissors, dissectors and graspers, into a cavity of the body at the surgical site. Examples of known surgical scopes are disclosed in patent documents bearing publication numbers US 4,491,865 A, US 5,325,847 A, JP 2002 291693 A, DE 10 2004 023866 B3 and US 7,435,218 B2. Surgery is performed while observing the site captured by a scope and displaying a live image feed on a video monitor for observation by the surgeon. As such, learning laparoscopic surgery is very difficult, as the surgeon does not observe the organs and tissues directly with the naked eye. Visual information is obtained indirectly via a monitor displaying a two-dimensional image. The loss of information when presenting a three-dimensional environment via a two-dimensional image is significant. In particular, depth perception is reduced when viewing a two-dimensional image as a guide for manipulating instruments in three dimensions. Furthermore, trocars are inserted through small incisions and rest against the abdominal wall. As a result, the manipulation of instruments/scopes is restricted by the abdominal wall, which has a fulcrum effect on the instrument/scope. Hence, hand-eye coordination skills are necessary and must be practiced in order to correlate hand motion with tool tip motion. The surgeon must also develop a set of core haptic skills because tactile sensation is diminished, as the surgeon cannot palpate the tissue directly by hand. The acquisition of all of these skills and more is a challenge in laparoscopic training and there is a need for scopes that are suitable for use in a training environment.

### Summary

According to the present invention, there is provided a simulated surgical camera scope as recited in Claim 1.

Many of the attendant features of the present invention will be more readily appreciated as the same becomes better understood by reference to the following description and considered in connection with the accompanying drawings in which like reference symbols designate like parts throughout.

### Brief Description of the Drawings

FIG 1 is a schematic of an optical train and terms of a surgical simulation camera scope in accordance with various embodiments of the present invention.
FIG. 2 is a side view of a surgical simulation camera scope in accordance with various embodiments of the present invention.
FIG. 3 is a top view of a surgical simulation camera scope in accordance with various embodiments of the present invention.
FIG. 4 is a cross-sectional view taken along line 4-4 of FIG. 3 of a surgical simulation camera scope in accordance with various embodiments of the present invention.
FIG. 5 is a partial cross-sectional view, detail 5 of FIG. 4, of the distal end of a surgical simulation camera scope in accordance with various embodiments of the present invention.
FIG. 6 is an exploded, top perspective view of a surgical simulation camera scope in accordance with various embodiments of the present invention.
FIG. 7 is a top perspective view of a sensor mount of a surgical simulation camera scope in accordance with various embodiments of the present invention.
FIG. 8 is a top perspective view of a sensor mount of a surgical simulation camera scope in accordance with various embodiments of the present invention.
FIG. 9 is an end view of a sensor mount of a surgical simulation camera scope in accordance with various embodiments of the present invention.
FIG. 10 is a cross-sectional view taken along line 10-10 of FIG. 9 of a sensor mount of a surgical simulation camera scope in accordance with various embodiments of the present invention.
FIG. 11 is an end view of a sensor mount of a surgical simulation camera scope in accordance with various embodiments of the present invention.
FIG. 12 is a cross-sectional view of a sensor mount of a surgical simulation camera scope in accordance with various embodiments of the present invention.
FIG. 13 is a side elevation view of a shaft, sensor assembly and lens assembly of a surgical simulation camera scope in accordance with various embodiments of the present invention.
FIG. 14 is a cross-sectional view taken along line 14-14 of FIG. 13 of a shaft, sensor assembly and lens assembly of a surgical simulation camera scope in accordance with various embodiments of the present invention.
FIG. 15 is a cross-sectional view taken along line 15-15 of FIG. 13 of a shaft, sensor assembly and lens assembly of a surgical simulation camera scope in accordance with various embodiments of the present invention.
FIG. 16 is a detail view of the distal end of a shaft, sensor assembly and lens assembly of FIG. 15 of a surgical simulation camera scope in accordance with various embodiments of the present invention.
FIG. 17 is a partial, sectional view of a shaft, sensor, lights and flexboard of a surgical simulation camera scope in accordance with various embodiments of the present invention.
FIG. 18 is a partial sectional view of a sensor assembly and lens assembly of a surgical simulation camera scope in accordance with various embodiments of the present invention.
FIG. 19 is a sectional end view of a surgical simulation camera scope in accordance with various embodiments of the present invention.
FIG. 20 is a top perspective view of a lens mount of a surgical simulation camera scope in accordance with various embodiments of the present invention.
FIG. 21 is bottom perspective view of a lens mount of a surgical simulation camera scope in accordance with various embodiments of the present invention.
FIG. 22 is a top view of a lens mount of a surgical simulation camera scope in accordance with various embodiments of the present invention.
FIG. 23 is a cross-sectional view taken along line 23-23 of FIG. 22 of a lens mount of a surgical simulation camera scope in accordance with various embodiments of the present invention.
FIG. 24 is a cross-sectional view taken along line 24-24 of FIG. 22 of a lens mount of a surgical simulation camera scope in accordance with various embodiments of the present invention.
FIG. 25 is a bottom view of a lens mount of a surgical simulation camera scope in accordance with various embodiments of the present invention.
FIG. 26 is a detailed view of detail 26 of FIG. 25 of a lens mount of a surgical simulation camera scope in accordance with various embodiments of the present invention.
FIG. 27 is a cross-sectional view taken along line D-D of FIG. 28 of a lens mount of a surgical simulation camera scope in accordance with various embodiments of the present invention.
FIG. 28 is an end view of a lens mount of a surgical simulation camera scope in accordance with various embodiments of the present invention.
FIG. 29 is a partial schematic of a lens and sensor arrangement of a surgical simulation camera scope in accordance with various embodiments of the present invention.
FIG. 30 is a perspective view of a pin of a surgical simulation camera scope in accordance with various embodiments of the present invention.
FIG. 31 is a top view of a pin of a surgical simulation camera scope in accordance with various embodiments of the present invention.

### Detailed Description

Generally, a simulation surgical camera scope is provided to assist in laparoscopic surgical skill training and simulation. The environment for training laparoscopic surgical skills can include a box trainer that is intended to simulate the human abdominal area. The trainer in various embodiments includes a penetrable cover that simulates an abdominal wall through which surgical instruments and scopes are inserted to access the simulated body cavity that houses artificial organs or skills training models upon which mock surgical procedures are practiced. Real surgical or surgical-grade scopes may be employed in a training environment. Surgical-grade scopes are high quality, calibrated, precision instruments, have very limited optical distortion, and provide enough light to completely illuminate the cavity. Surgical-grade scopes however are also very expensive costing thousands of dollars. Because surgical-grade scopes are expensive and can be easily mishandled, damaged, and scratched by less experience users, there is a need for training scopes for use in a training environment. In addition, less expensive scopes and scopes that are more portable are needed, not only in a training environment, but also, in a marketing environment to showcase instruments and demonstrate new procedures on the go. Scopes of the same caliber as surgical-grade scopes are not needed in a training or marketing environment and may be too cumbersome and expensive to port. In addition, because budgets for surgical training and simulation centers are limited, there is a need for inexpensive yet effective scopes designed with training purposes in mind.

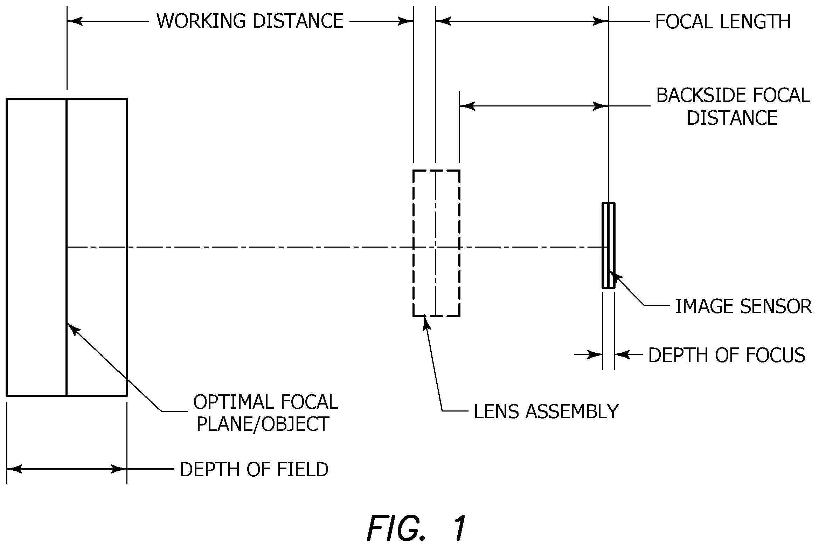

The optical performance of a simulation surgical scope should be similar to surgical-grade scopes with respect to certain characteristics. These characteristics include, but are not limited to, working distance, depth-of-field, field-of-view, image color and/or image quality. Some of these characteristics are schematically depicted in FIG. 1 for readability of the description and not a suggestion of prior art or admission of such. Image quality can include the sharpness of the video images in terms of pixel count resolution and the corresponding frames per second (fps) of the video feed. The simulation surgical scope in accordance with various embodiments provides an image quality that is approximately 640x480 to 720x1024 at approximately 24-60 fps. With respect to image color, the simulation surgical scope in accordance with various embodiments provides an approximate to the actual colors of an object when observed with the naked eye. Hence, the lighting employed at the tip of the training scope should not distort the colors of the organ models so that the realism of the simulation is not compromised. The working distance, which is the distance from the lens to the optimal focal plane is shown in FIG. 1, which is the plane of best focus. Still referencing FIG. 1, the depth-of-field is defined as the distance away from the optimal focal plane in both directions in which the image is still in focus. The depth-of-field should be as wide as possible. The field-of-view is defined as the area in the displayed image at the working distance. These and other characteristics work together to make for an image that is suitable for a simulation surgical scope in accordance with various embodiments of the present invention. Also, these parameters can provide the image, size, magnification, and/or other similar characteristics that correspond to a surgical-grade scope so that the trainee is not surprised when switching from the simulation surgical scope to using a surgical-grade scope.

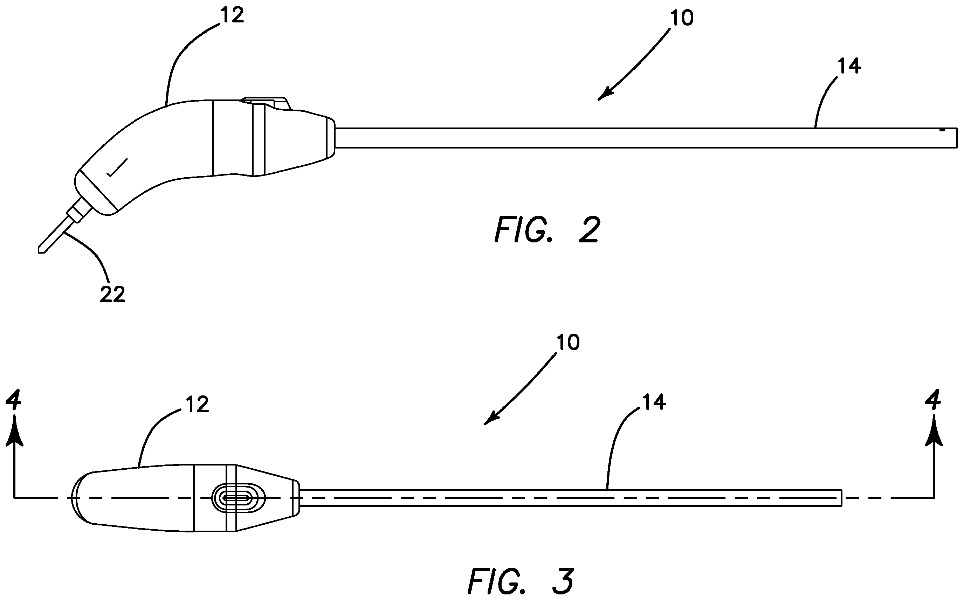

With reference to FIGs. 2-6, the scope 10 in accordance with various embodiments includes a handle 12 connected to an elongated shaft 14. A sensor assembly 16 and lens assembly 18 are located inside the scope 10 and connected to the elongated shaft 14. The sensor assembly 16 is connected to a controller printed circuit board assembly (PCBA) 20 located inside the handle 12. The scope 10 is connectable to a computer or video monitor wirelessly or via a cable 22, such as a USB cable, and configured to display the captured video image.

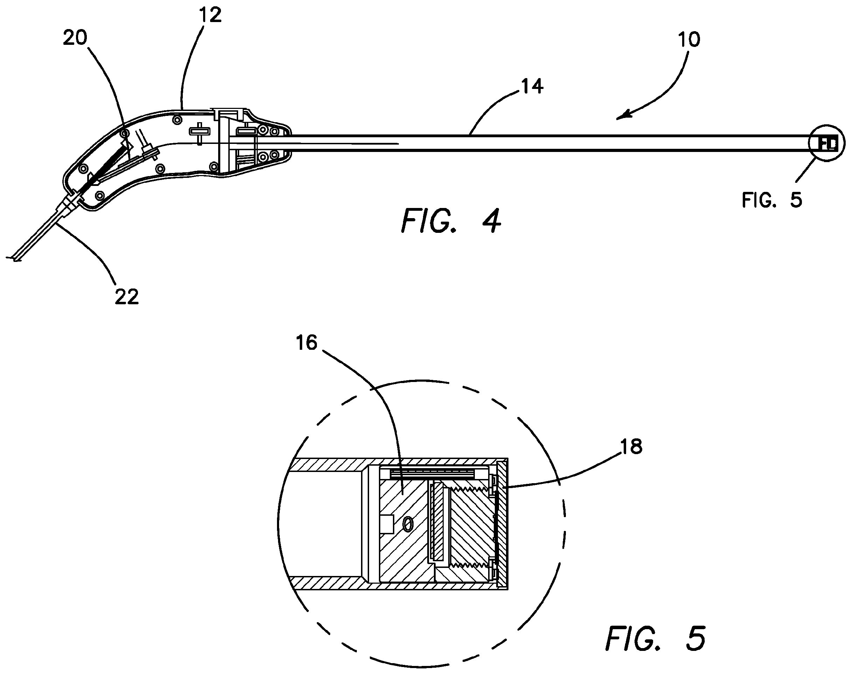

With particular reference to FIG. 4, the handle 12 is a two-piece, clamshell design that defines an interior and houses the controller PCBA 20. The PCBA is connected to the sensor assembly 16 and to an output cable 22 that is in turn connected to a computer or video monitor in order to display a video image captured by a sensor and processed by a microcontroller on the controller PCBA. The elongated shaft 14 is connected to the distal end of the handle 12. In various embodiments, the handle 12 is provided with one or more buttons connected to a microcontroller on the PCBA to control the power and lighting of the scope 10. The lumen of the elongated shaft 14 opens to the interior of the handle 12 in order to connect the sensor assembly 16 to the PCBA 20.

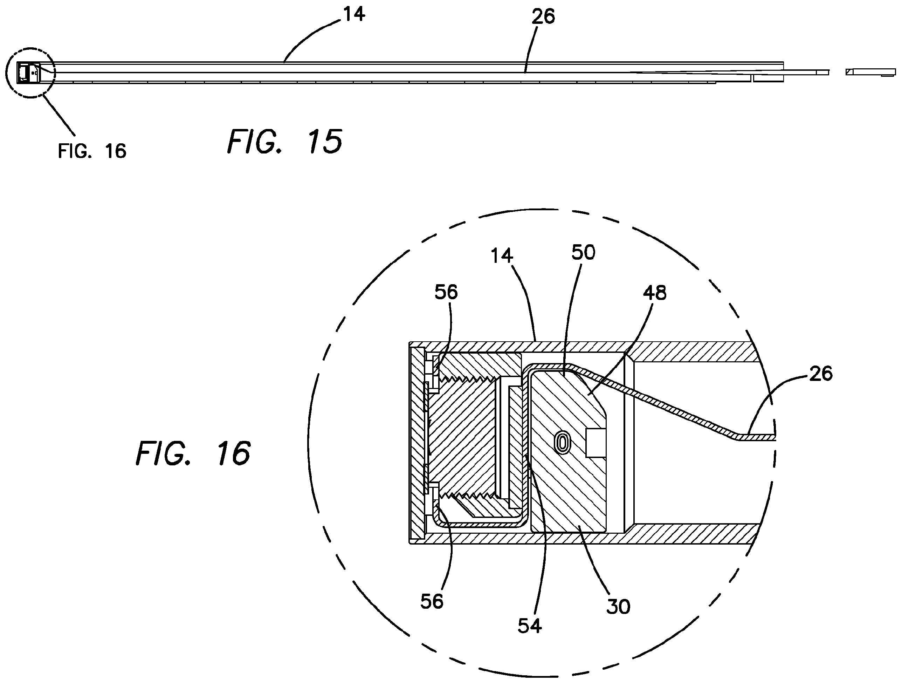

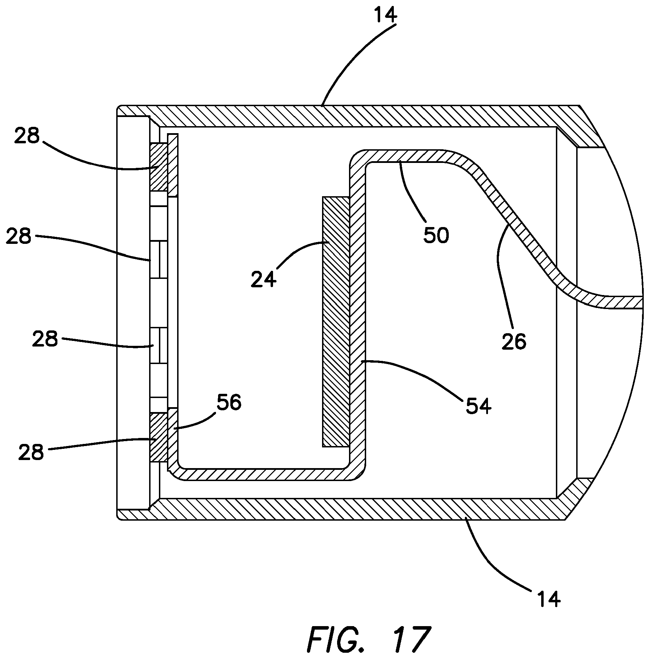

The elongated shaft 14 includes a sidewall having a cylindrical shape defining a lumen. The shaft 14 includes a proximal end and a distal end. The proximal end of the shaft 14 is connected to the handle 12 and includes a proximal opening such that the lumen of the shaft 14 opens to the interior of the handle 12. The distal end of the shaft 14 includes a distal opening. The diameter of the shaft 14 is approximately between 3mm to 15mm. The outside diameter is sized to fit inside a correspondingly sized trocar. For example, if the outside diameter of the shaft 14 is 10mm, the inside diameter of the trocar is greater than 10mm. The inner diameter of a 10mm shaft 14 is approximately 8mm. Hence, in such a variation, the diameter of the sensor is less than 8mm so that it fits inside the shaft. Not only is the outer diameter sized to fit inside a corresponding trocar, but also, the inner diameter of the shaft 14 is sized to receive a sensor inside the shaft 14. In one variation, the inner diameter of the shaft 14 has a larger inner diameter at the distal end relative to the proximal end of the shaft 14 in order to house a larger sensor. For example, a shaft 14 with an inner diameter of approximately 8mm is machined to be larger, approximately 9mm at the distal end of the shaft 14 in order to fit a 9mm sensor. Hence, the inner diameter of the shaft is stepped to a larger diameter at the distal end relative to the inner diameter at the proximal end as can be seen in FIGs. 16-17. The length of the shaft 14 is approximately 30-36cm. The length from the handle 12 to the distal end of the shaft 14 is approximately 33cm.

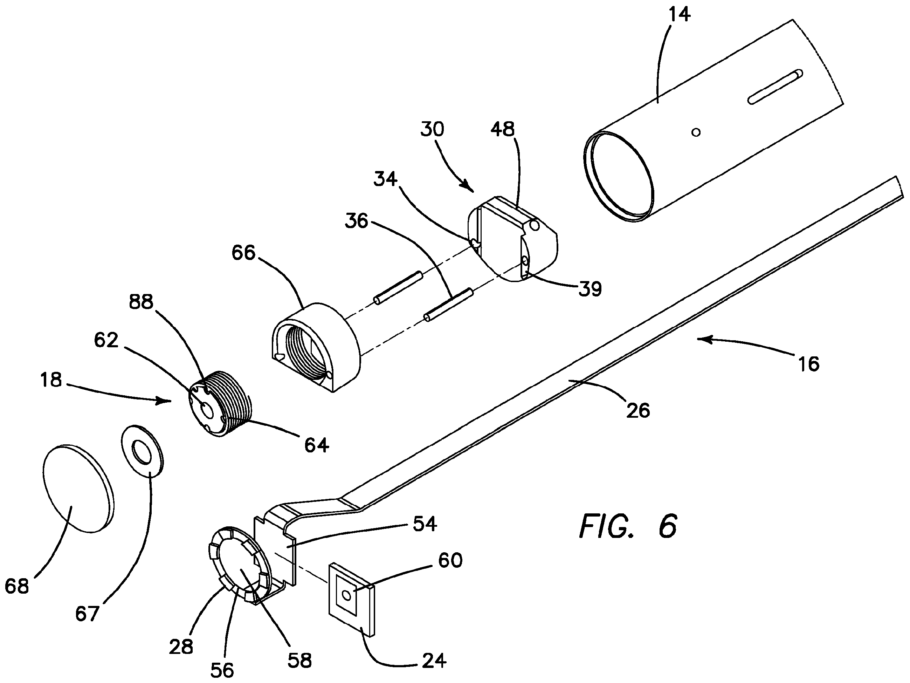

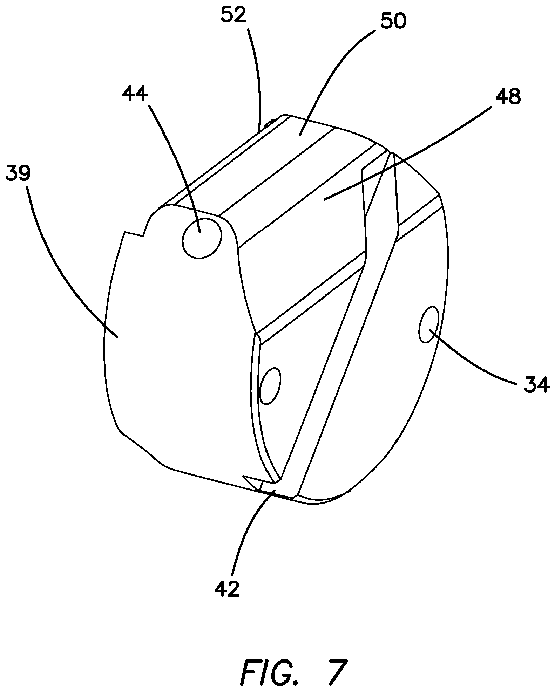

With additional reference to FIGs. 6-13, the sensor assembly 16 will now be described in greater detail. The sensor assembly 16 includes a sensor 24 and lights 28 mounted on an electrical connector 26. The sensor assembly 16 further includes a sensor mount 30. The sensor mount 30 is connected to the elongated shaft 14 via an indexing, mounting pin 32.

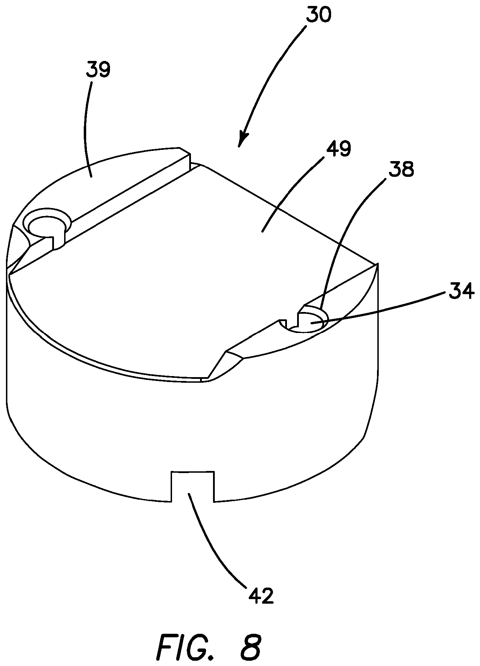

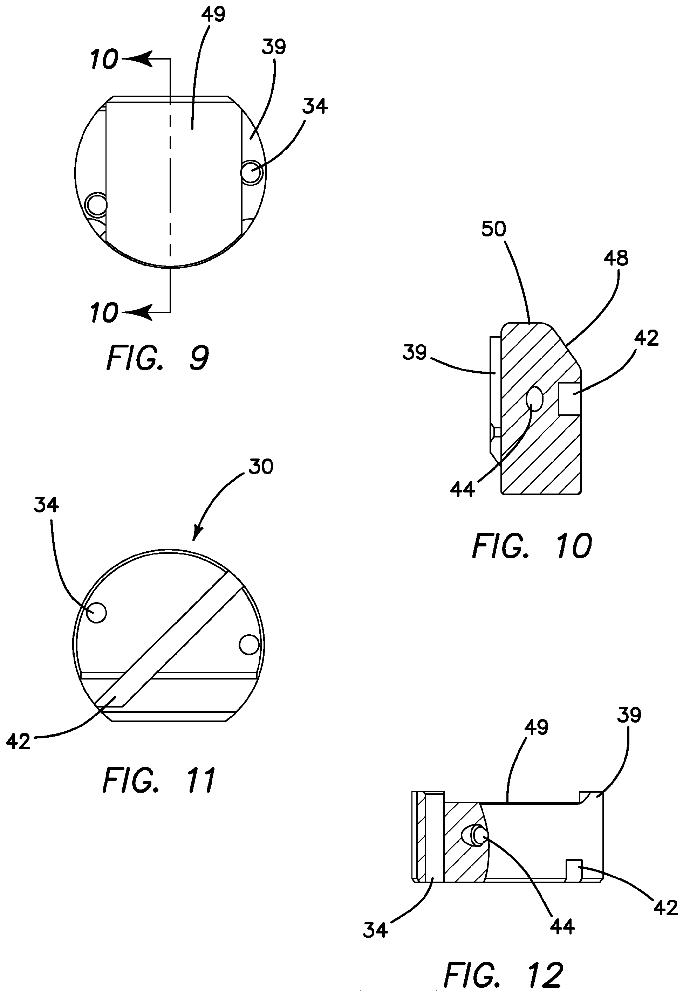

With continued reference to FIGs. 6-13, the sensor mount 30 will now be described in greater detail. The sensor mount 30 is sized and configured to provide a backing and support for the sensor 24 and connector 26. The sensor mount 30 is partially cylindrical in shape and is sized to fit closely within the lumen of the elongated shaft 14. The sensor mount 30 has a proximal end and a distal end interconnected by a sidewall. Two pin holes 34 are provided extending parallel to the longitudinal axis of the shaft 14. The pin holes 34 extend between the proximal end and the distal end and are sized and configured to receive a pin 36' or dowel 36 in each pin hole 34. Each pin hole 34 includes a beveled entry 38 at the distal end of the pin hole 34 as can be seen in FIG. 8. The beveled entry 38 is a ramped or tapered entryway to facilitate insertion of a pin 36' or dowel 36 into the pin hole 34. In various embodiments, the distal end of the pin hole 34 includes a length 40 of the pin hole 34 having a diameter that is slightly larger than the diameter of the pin hole 34 at the proximal end as can be seen in FIG. 12.

In various embodiments, the dowel to pin hole interface is a slip fit arrangement, e.g., the diameter of the hole 34 is larger than dowel 36. This locates the sensor mount and the lens mount to each other in the radial directions, but allows movement in the axial direction. When the assembly is ready, glue is placed on the dowel and hole interface on the backside of the sensor mount to prevent the sensor mount from separating from the lens mount. It should be noted that care is taken before the application of the adhesive to ensure that the sensor mount and lens mount are aligned both radially and axially before permanently affixed together. It has been found that affixing the alignment of the sensor and lens mount in one direction, e.g., radial, first and then adjusting the sensor and lens mount in another direction, e.g., axial, ensures optimal alignment of the sensor and lens mount. It has also been found that allowing movement or adjustment of the sensor and lens mounts axially provides a precise placement of the sensor relative to the lens to ensure the sensor is within the depth of focus of the lens. Placement of the sensor outside of this range degrades image quality or inoperability of the scope. Likewise, any radial and skewed placement of the senor also degrades image quality. Additionally, the relaxed or slip fit arrangement allows the coupling of the sensor and lens mount to be precise by avoiding friction and in particular friction that can cause sudden axial movement as the sensor and lens mounts are coupled together. Sudden axial movement or imprecise axial coupling of the sensor and lens mounts together can cause damage to the sensor and thus cause the scope to be inoperable.

Another pin hole 44 that is traverse to the longitudinal axis of the elongated shaft 14 is provided in the sensor mount 30 and extends through the sensor mount 30 between the sidewall. As can be seen in FIG. 9, the traverse pin hole 44 also includes an enlarged diameter portion 46 to facilitate insertion of a pin 32 as well as alignment and setting of the sensor mount 30 with respect to the elongated shaft 14. The pin 32 is longer than the dowel 36 or pin 36' employed in pin holes 34.

The proximal end of the sensor mount 30 includes a channel-like groove 42 sized and configured to receive an instrument such as a flat-head screwdriver. The groove 42 assists in the installation of the sensor mount 30 within the elongated shaft 14. During assembly, an instrument can be inserted into the groove 42 in order to help hold and move the sensor mount 30 into the lumen of the elongated shaft 14, hold the sensor mount 30 in position inside the elongated shaft 14, rotate the sensor mount 30 with respect to the elongated shaft 14, align the sensor mount 30 with respect to corresponding pin holes formed in the elongated shaft 14, and/or generally hold the sensor mount 30 stationary with respect to elongated shaft 14. The proximal end of the sensor mount 30 is substantially parallel to the distal end of the sensor mount 30. The proximal end of the sensor mount 30 also includes an angled surface 48 interconnected with a flat portion 50 of the sidewall. The angled surface 48 together with the flat portion 50 provide a landing and pathway for the connector 26 extending proximally inside the elongated shaft 14 to the PCBA 20 located in the handle 12. The distal end of the sensor mount 30 includes a bevel 52 at the intersection of the distal end with the flat portion 50 of the sidewall. The bevel 52 helps to provide a smooth bend in the connector 26 to prevent a sharp bend and associated stress concentrations in the connector 26.

In various embodiments, the distal end of the sensor mount also includes raised surfaces 39 disposed on either side of a middle, flat cavity or surface 49. The sensor and connector 26 are positioned on the middle, flat surface 49 of the sensor mount 30. It has been found that due to the limitations in size and dimensions of the distal end of the scope and the need for the precise placement of the sensor relative to the lens, the sensor is often crushed or damaged between the sensor and lens mounts. As such, raised surfaces 39 act as a hard stop to prevent crushing of the sensor between the sensor mount and lens mount. Thus, the dimensions and tolerance of the raised surfaces relative to the middle surface are such that when the sensor mount and lens mount are coupled or connected together with the sensor and connector between them, the sensor is not crushed. Additionally, there is a gap of 0.038mm (0.0015") or less between the flexboard assembly and the sensor mount and in various embodiments that is also accommodated by the raised surfaces. Accordingly, in accordance with various embodiments, the sensor and/or lens mount are configured to ensure that the spacing between the lens and sensor mounts is greater than the thickness of the sensor and the flexible printed circuit board and yet smaller than or no greater than the depth of focus range of the lens thereby ensuring the sensor is always located within the depth of focus, ensuring image quality is maintained and the sensor is not damaged. As such, in various embodiments, the height of the raised surface relative to the middle surface of the sensor mount is not greater than the depth of focus range of the lens.

In accordance with other various embodiments, the light sensing portion of the senor is indexed to the lens mount to keep the crucial distance between the sensor and lens consistent. When assembling, the sensor mount without raised surfaces and the lens mount are brought together with less pressure than would crush the sensor. Force or pressure gauges or other similar force or pressure limiting components would be utilized to ensure that the threshold crush pressure of the sensor is not exceeded. Once in place, i.e., the sensor is located within the depth of focus of the lens, the mounts are affixed together, e.g., glued to the pins, preventing axial movement of the mounts relative to the sensor. In various other embodiments, the raised surfaces extends further from the middle surface, i.e., have a greater height or are larger in the axial direction, resulting in a bigger gap or spacing between lens and sensor mounts when assembled. To index the sensor to the lens mount, a mechanical spring or a material with spring like attributes, like silicone, is placed in the gap or spacing that is created by the raised surfaces, thus taking up the resulting gap of having the raised surfaces further from the middle, flat surface. In various embodiments, the sensor can be indexed, with a set screw coming from the proximal end of the sensor mount or similarly through the use of shim stock, to push the sensor against the lens mount.

In various embodiments, the connector 26 is a flexible circuit board or flexboard 26. The flexboard 26, in one embodiment, is approximately 17 inches long, approximately 0.10 inches wide and approximately 0.015 inches thick. The flexboard 26 is a connective device that provides an electrical connection between electrical components. The flexboard 26 is connected to the sensor 24 and lights 28, extends along the length of the shaft 14 and connects to the PCBA 20 inside the handle 12. The flexboard 26 reduces wiring errors during assembly, reduces assembly time and costs, eliminates mechanical connectors and provides design flexibility including highly complex configurations and provides a support for surface mounted devices. The flexboard 26 can be made to conform to a desired shape and flex during use and installation. The flexboard 26 is substantially planar and is connected at its proximal end to the PCBA 20 inside the handle 12. The flexboard 26 extends distally inside the shaft 14 as can be seen in FIG. 15. The flexboard 26 will twist approximately 90 degrees within the shaft 14 to facilitate connection with the sensor assembly 16 at the distal end and the PCBA 20 at the proximal end.

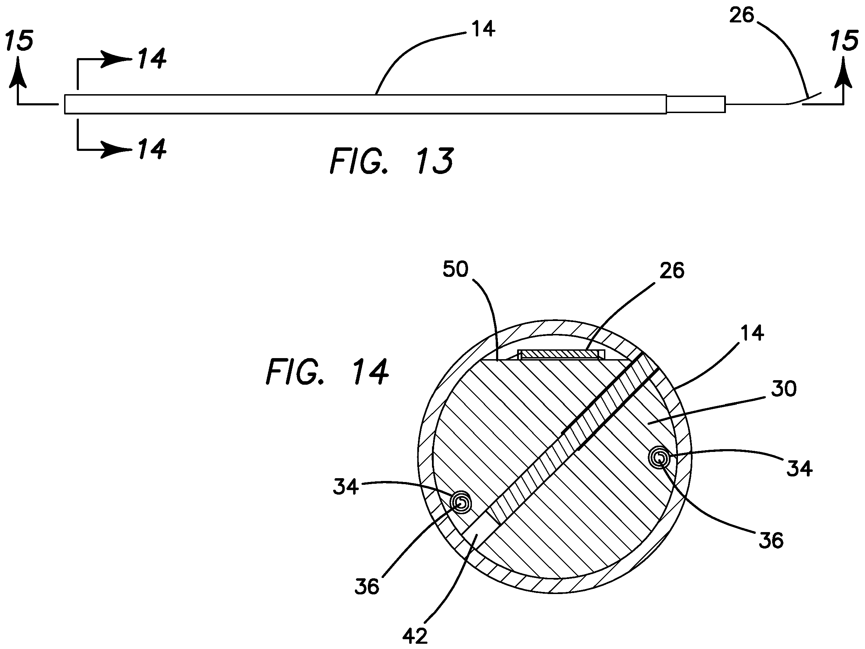

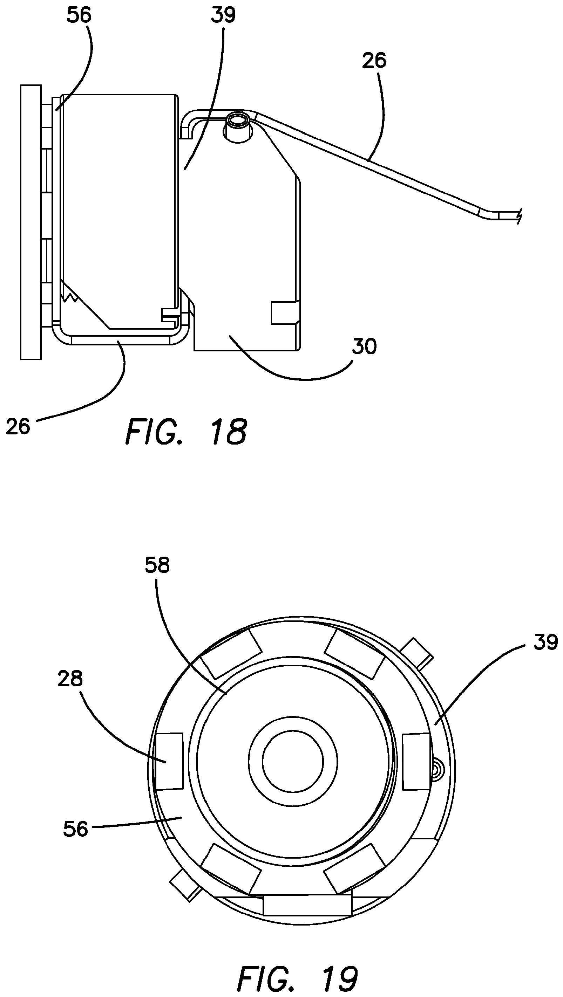

With particular reference to FIGs. 16 and 18, the flexboard 26 makes contact with the angled surface 48 of the sensor mount 30. The flexboard 26 is directed to pass over the flat portion 50 of the sidewall. The flat portion 50 advantageously reduces the diametrical distance of the sensor mount 30 to accommodate the thickness of the flexboard 26 as can be seen in FIG. 16 and in the FIG. 14. The flexboard 26 may be adhered with adhesive to the flat portion 50. From the flat portion 50, the flexboard 26 turns to extend along the distal end of the sensor mount 30. The flexboard 26 in one embodiment may be adhered to the distal end of the sensor mount 30. The distal end and proximal end of the sensor mount 30 are perpendicular to the longitudinal axis of the shaft 14. The flexboard 26 is also substantially perpendicular to the longitudinal axis of the shaft 14 along the distal end of the sensor mount. This perpendicular and flat portion of the flexboard 26 along the distal end of the sensor mount 30 is called a sensor location 54. It is at this location that the sensor 24 is connected to the flexboard 26 as can be seen in FIGs. 16-17. The sensor location 54 has an enlarged flat area compared to the flexboard 26 that is proximal to the sensor location. After the distal end of the sensor mount 30, the flexboard 26 makes an approximately 90 degree bend and extends distally before bending approximately 90 degrees to form a lighting or LED location 56 that is also approximately perpendicular to the longitudinal axis of the shaft 14. The lighting or LED location 56 of the flexboard 26 is where the lights/LEDs 28 are connected to the flexboard 26. The LED location 56 is flat and includes a central opening 58 as can be seen in FIGs. 6 and 19. The central opening 58 permits unobstructed and illuminated viewing via the lens assembly 18. In one variation, the lights 28 are a plurality of LEDs connected to the flexboard 26 with electrically conductive solder or adhesive and arranged in a circle around the central opening 58 and around the lens prior to assembly with the rest of the scope components. The flexboard 26 forms an S-shaped pathway as can be seen in FIGs. 16-18.

The flexboard 26 includes electrical contacts for the LEDs 28 and image sensor 24 and traces that allow power and data to be transmitted to the controller PCBA 20. The flexboard 26 also locates the LEDs 28 and image sensor 24 for assembly with the rest of the components in the shaft 14. The flexboard 26 eliminates the use of wires, is flat and smaller than an equivalent wire bundle it is replacing and can be designed and configured to meet the confines of the shaft dimension and assembly components. Furthermore, due to the sensor 24 being placed at the distal end of the scope and the controller PCBA 20 being positioned at the proximal end of the scope and the speed and/or amount of data being transmitted, the signal integrity between the components can degrade. However, the flexboard 26, in various embodiments, being a single monolithic component ensures that signal integrity of data transfer from the image sensor 24 is not compromised. In various embodiments, the flexboard includes, integrated or attached, a metallic layer, e.g., a copper only layer, to ensure signal integrity is maintained. The metallic layer is sized and dimensioned in various embodiments to shield the flexboard from outside electrical noise that can interfere with the signals transmitted through the traces on the flexboard, especially the high speed data lines. The flexboard 26 is sufficiently flexible to navigate the S-shaped pathway making multiple approximately 90-degree bends to meet the design of the sensor assembly 16 and lens assembly 18.

A ring of LEDs 28 are employed in the sensor assembly 16 and arrayed around the lens assembly as can be seen in FIG. 19. The LEDs 28 serve to illuminate the target object and surgical field. Approximately, 4-8 white LEDs 28 are used depending on the brightness required for a particular application or to allow use in all conditions including confined procedures such as transanal minimally invasive surgery (TAMIS) which may require more lighting. The LEDs may be selected to have an appropriate color temperature, or tinted, e.g., via tinted glass 67, to optimize color matching.

The sensor 24, in one embodiment, is a CMOS image sensor such as one produced by OmniVision Technologies, Inc. It is a ¼ sensor sized to fit within a 10mm shaft 14. The sensor 24 supports video quality maximums of 720 pixels at 60 fps and has a resolution of 640x480 characteristic of the VGA hardware at 90 fps. The sensor 24 supports a video output format of YUV422 and has inter-integrated circuit communications to communicate with a microprocessor and has a camera serial interface 2 (CSI-2) mobile industry processor interface (MIPI) for the video communications. Sensors that would be small enough to fit within a 5mm tube do not have the associated microprocessor, electronics and demosaicing algorithm to output in YUV422. Therefore, a separate circuit board would be needed to support a microprocessor and associated algorithm external to the sensor. The sensor 24 in various embodiments has a smaller active image area 60 comprising the array of photo sites. The sensor 24, which is not limited to a CMOS type sensor, is adhered with solder or electrically conductive adhesive to the flat sensor location 54 on the flexboard 26.

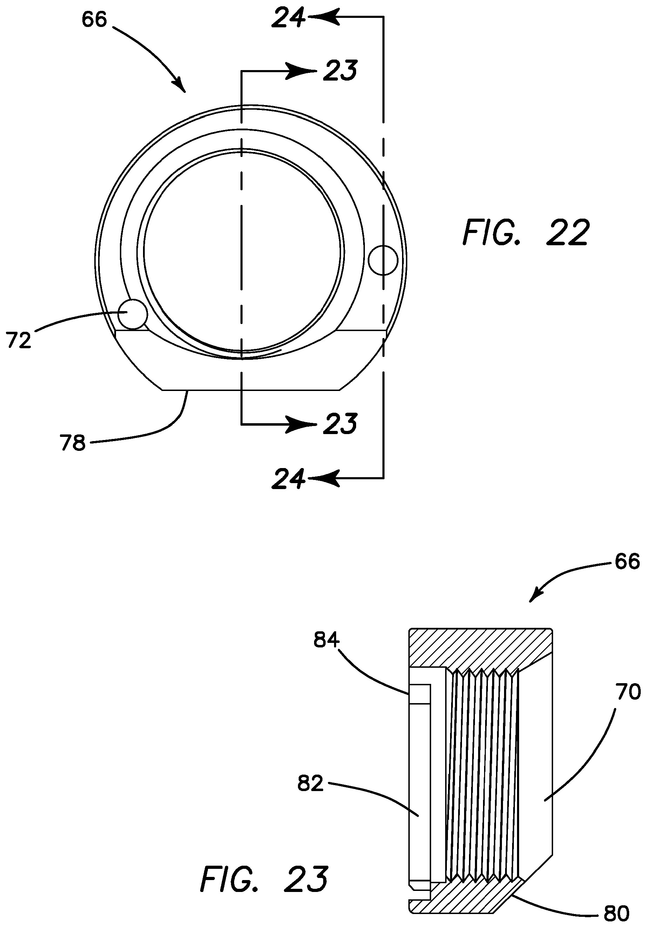

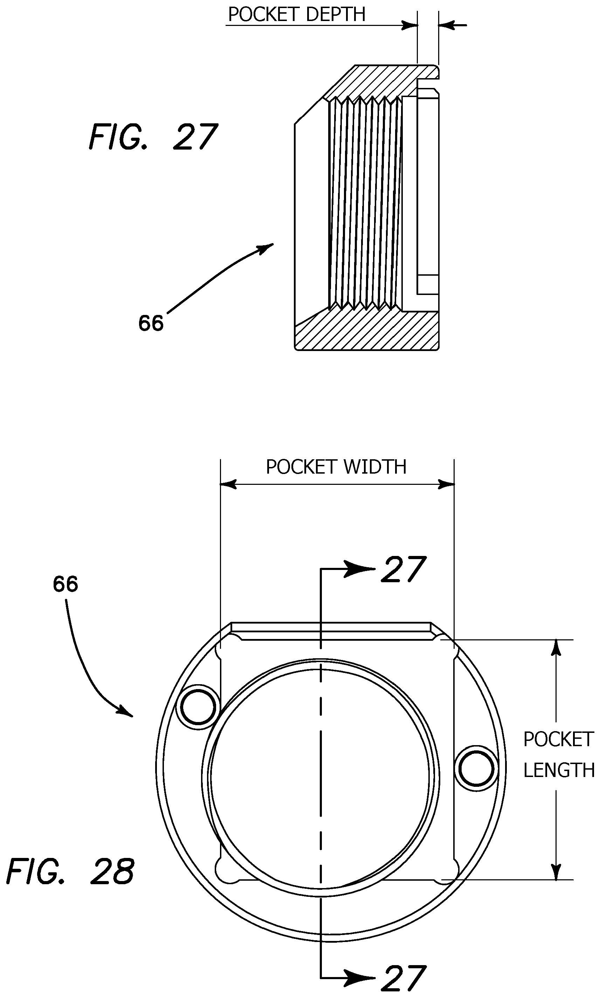

With reference to FIGs. 6 and 20-28, the lens assembly 18 will now be described. The lens assembly 18 in accordance with various embodiments includes a lens 62, a lens housing 64, a lens mount 66, a tinted glass 67, a cover glass 68 and/or combinations thereof. The lens 62 in various embodiments may include one or more lenses and optical elements. In various embodiments, the lens 62 includes an optical train having four lenses and an infrared filter. The lens 62 has a 3.8mm focal length and is aspheric and achromatic. This configuration provides a working distance of approximately 50-70mm, a depth-of-field of approximately 90-150mm and a field-of-view of 60-65 degrees. Also, this configuration minimizes optical distortion while keeping costs down. The optical distortion is limited to the edges of the optical image. Since the diagonal dimension of the image area 60 of the sensor 24 is smaller than the diagonal of the focused optical image hitting the sensor, the distorted edges are not picked up by the sensor giving a clean non-distorted image on the monitor. The lens 62 is connected to and located inside the lens housing 64. The lens housing 64 is cylindrical in shape and includes an outer surface that is threaded. The threads are configured for threaded engagement with the lens mount 66. . The lens housing 64 further includes one or more notch, socket or the like 88 configured for receiving a distal end of a correspondingly shaped instrument. The instrument is inserted into the notch/socket 88 of the lens housing 64 in order to rotate the lens housing 64 with respect to the lens mount 66 for optically tuning the lens assembly.

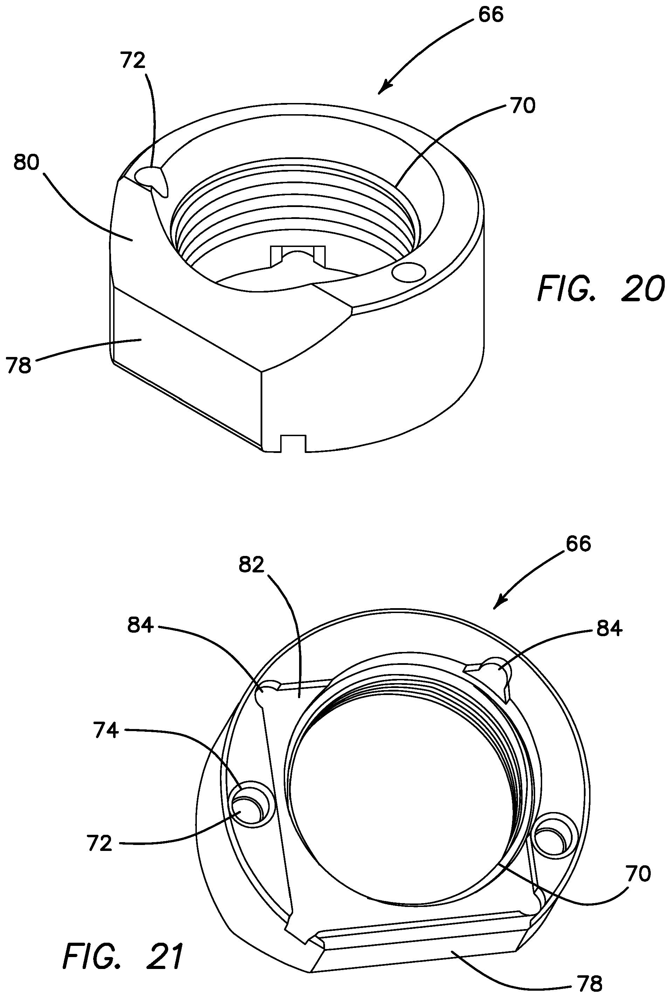

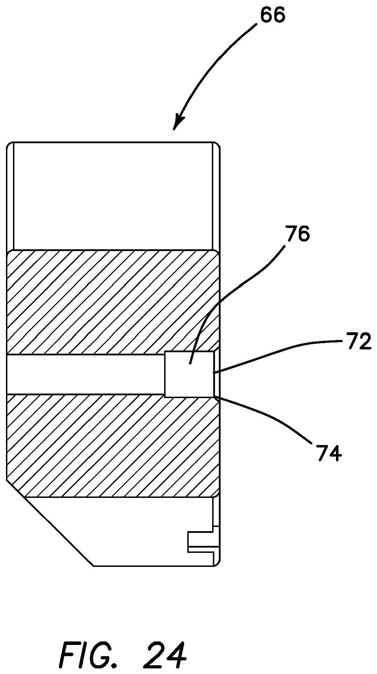

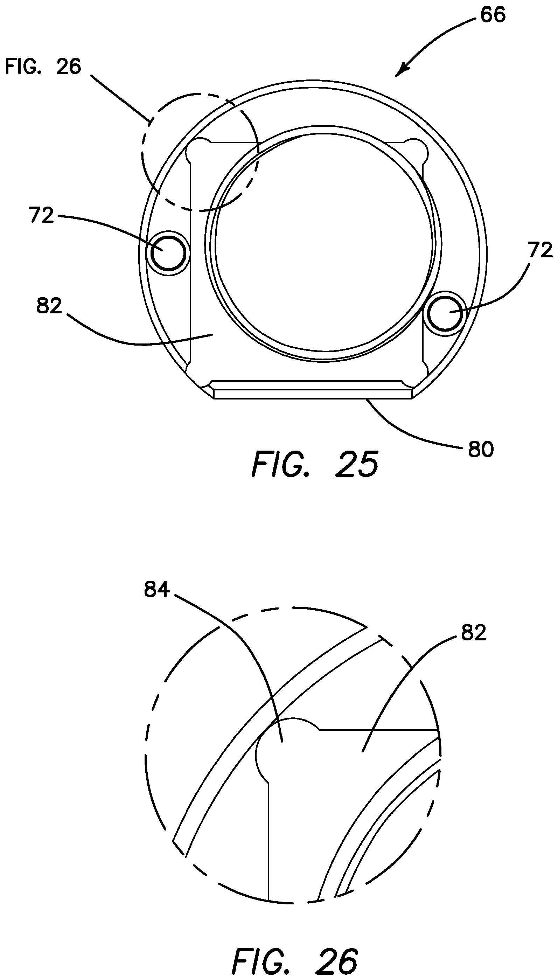

With particular reference to FIGs. 20-28, there is shown the lens mount 66. The lens mount 66 includes a proximal end and a distal end interconnected by a sidewall. The lens mount 66 includes a central lumen 70 extending between an opening at the proximal end and an opening at the distal end. The lens mount 66 is partially cylindrical and is sized to fit closely within the lumen of the elongated shaft 14. The central lumen 70 includes a threaded inner surface for engaging the threads on the outer surface of the lens housing 64 such that, when threaded into the lens mount 66, the lens 62 moves along the longitudinal axis. The lens mount 66 includes two pin holes 72 that extend between the proximal end and the distal end. The pin holes 72 are sized and configured to receive a dowel 36 or pin 36' in each pin hole 72. Each pin hole 72 includes a beveled entry 74 at the proximal end of the pin hole 72 as can be seen in FIGs. 21 and 24. The beveled entry 74 is a ramped or tapered entryway of greater diameter to facilitate insertion of a dowel 36 or pin 36' into the pin hole 72. A portion 76 of the length of the pin hole 72 has a larger diameter relative to the remaining length of the pin hole 72 that is distal to the larger diameter portion 76 as can be seen in FIG. 24. The beveled entry 74 has a diameter larger than the diameter of portion 76, which has a diameter larger than the rest of the pin hole 72. The enlarged diameter portion 76 allows the dowel 36 or pin 36' to be located within the enlarged diameter portion 76 before deeper insertion into the pin hole 72 allowing for the lens mount 66 to be located and set in position within the elongated shaft 14. The sidewall of the lens mount 66 includes a flat portion 78. The flat portion 78 transitions to a bevel 80 at the intersection of the flat portion 78 with the distal end. Together, the flat portion 78 and bevel 80 provide room and a smooth transition for the flexboard 26 as it bends approximately 90 degrees and extends in juxtaposition to the flat portion 78. The proximal end of the lens mount 66 includes a recess 82 that is also called a pocket. The recess 82 is a surface that is a planar notched depression into the proximal end in the shape of the sensor 24 and sized slightly larger than the sensor 24 in order to form a receiving location for the sensor 24. The recess 82 encompasses the proximal opening of the substantially central lumen 70. The recess 82 is slightly offset and not coaxial with the proximal opening of the substantially central lumen 70 in order to align the active image area 60 of the sensor 26 with the axis of the optical train of the lens 62. The shape of the recess 82 matches the shape of the sensor 24. The recess 82 is a square, rectangle, parallelogram or other shape. The recess 82 includes radiused corners 84 with radii on the exterior of the corner as shown in FIGs. 25-26. Not all of the corners are radiused as at least one corner is outside the sidewall perimeter. The radiused corners 84 allow the sensor to closely fit within the recess 82 such that at least a portion of the perimeter edges of the sensor 24 is in juxtaposition with the four sides of the recess 82. In one variation shown in the figures, not all of the four sides of the recess 82 are straight. At least two sides of the recess 82 are curved sharing the curvature of the lumen opening at the proximal end as can be seen in FIG. 25. The depth of the recess 82 or pocket depth is approximately 0.61mm (0.024 inches). When a sensor 24 is located inside the recess 82 translation of the sensor 24 laterally with respect to the longitudinal axis is prevented.

Still referencing FIG. 6, the lens assembly 18 includes a cover glass 68. The cover glass 68 is a glass disc that covers the distal opening of the elongated shaft 14. The cover glass can be made of sapphire crystal to prevent scratching, Gorilla Glass, alkali-aluminosilicate sheet glass, to prevent impact and coated borosilicate to reduce reflections depending on which properties of the glass are more important. For example, sapphire crystal protects against accidental scratching but it is more brittle and can break on impact. Gorilla Glass resists impacts but can be scratched. Coated borosilicate can mitigate reflections of the LEDs into the sensor. The cover glass 68 prevents damage to the internal components of the elongated shaft 14 especially the lens since it can scratch easily and is located at the distal tip. The cover glass 68 also prevents particulate matter from migrating into the elongated shaft 14 and obscuring the lens.

In accordance with various embodiments, to assemble the scope 10, two pins 36 or dowels 36' are inserted into the two pin holes 72 in the lens mount 66. In various embodiments, only the tapered portion 86 of pins 36 are inserted into the larger diameter portion 76 of each pin hole 72. The sensor mount 30 is aligned to bring the opposite ends of the pins 36 into the enlarged portion 40 of pin holes 34 formed in the distal end of the sensor mount 30. A pin alignment fixture, in various embodiments, that holds the sensor mount 30 and the lens mount 66 in position is used. The pins 36 that are partially inserted in their respective pin holes 34, 72 keep the sensor mount 30 and the lens mount 66 spaced apart for further assembly and alignment. The sensor 24 is soldered to electrically connect the sensor 24 to the flexboard 26. A portion of the flexboard 26 with the attached sensor 24 is then positioned between the sensor mount 30 and the lens mount 66 while the sensor mount 30 and lens mount 66 are held in a spaced-apart position by the alignment fixture. The sensor 24 is aligned and placed inside the recess 82 formed in the proximal end of the lens mount 66. The sensor mount 30 and lens mount 66 are coupled together with the distal end of the sensor 24 located in the pocket 82 and facing the lens 62 while the proximal end of the sensor 24 is attached to the sensor location 54 of the flexboard 26. The sensor location 54 is planar and substantially perpendicular to the longitudinal axis of the elongated shaft 14. The proximal end of the sensor location 54 of the flexboard 26 is supported by the distal end of the sensor mount 30. The flexboard 26 is then bent around the flat portion 50 of the sidewall and bevel 52 of the sensor mount 30. Distally, the flexboard 26 is bent under the flat portion 78 and around the bevel 80 of the lens mount 66.

In various embodiments, an optical system, e.g., the sensor mount 30 and lens mount 66 together with flexboard 26, sensor 24 and pins 36 are inserted into the elongated shaft 14. A long screwdriver or the like is used to engage the groove 42 at the proximal end of the sensor mount 30. The traverse pin hole 44 is aligned with an opening in the shaft 14 and a pin 32 is inserted into the pin hole 44 to lock the sensor mount 30 and associated lens mount 66 in position. The indexing pin 32 not only prevents longitudinal translation of the assembly, but also, prevents rotation of the assembly around the longitudinal axis of the shaft 14.

In accordance with various embodiments, the elongated shaft 14 is connected to one side of the handle 12. A receiving slot on the elongated shaft 14 is aligned with a tab formed on the inside of the handle 12. A clamp is positioned over the shaft 14 and tightened to permit movement of the shaft 14 with respect to the handle 12. The shaft 14 is permitted to rotate in order to ensure that the horizon line of an image captured by the scope 10 is level, horizontal with respect to a vertical orientation of the handle 12. The flexboard 26 and other wiring are connected to the PCBA 20 to create a functioning scope. To electrically connect the flexboard 26 to the PCBA 20, the flexboard 26 is attached by a 30-pin connector to the controller PCBA. The controller PCBA 20 has a CX3 microcontroller that supports YUV422 image format, provides I²C communications and supports MIPI Camera Serial Interface 2. The controller PCBA 20 also provides USB communication out to a monitor, regulates five voltage levels, and provides three different clock speeds for the microcontroller and image sensor. Firmware was written to provide the USB communication and to set the settings for the image sensor to meet the image requirements referred to previously. The scope 10 is then fitted with a USB cable that outputs the image to a video display, laparoscopic trainer or monitor.

In accordance with various embodiments, the shaft 14 and one side of the handle 12 is placed into an optical alignment fixture. The fixture, in various embodiments, has two towers and a toggle clamp. A target is illuminated and the scope 10 is connected to a computer monitor in order to observe an image of the target. The shaft 14 is rotated with respect to the handle 12 until the horizon becomes level, horizontal and then the toggle clamp is used to fix the position of the shaft 14. The handle 12 is then adjusted, rotated with respect to the shaft 14 until the handle 12 is vertical. The tube clamp is then tightened completely.

In various embodiments, after the assembly of the handle 12 is completed, the handle 12 and shaft 14 are placed into a press fixture. The other side of the handle clamshell is aligned in the fixture and the two halves of the handle 12 are pressed together to complete the handle and shaft assembly.

In accordance with various embodiments, the scope 10 is optically tuned by placing the scope into an optical alignment fixture. An optical target is provided and mounted into a slider movable along a rail in front of the distal end of scope 10 along the longitudinal axis of the shaft 12. The target is illuminated. The target is moved and aligned with the working distance of the lens. Then, an instrument is used to engage the socket 88 at the distal end of the lens housing 64 in order to rotate the lens housing 64 with the lens 62 inside. As the lens housing 64 is rotated, it is threadingly moved proximally or distally along the longitudinal axis with respect to the lens mount 66 to bring the image into focus. The position of the lens housing 64 with respect to the lens mount 66 is adjusted along the longitudinal axis until a middle "0" target is in acceptable focus. Focus should be acceptable through a depth-of-field range of approximately 140 mm with approximately 1/3 of that distance in front of the optimal focal plane and approximately 2/3 of that distance in back of the optimal focal plane. With the target image in focus, rotation of the lens housing 64 and tuning is finished.

After the scope 10 is optically tuned, in accordance with various embodiments, the cover glass 68 is attached to the shaft 14. The cover glass 68 is placed against the ring of LEDs connected to the flexboard 26. The cover glass 68 is then glued at four equally spaced locations to the shaft 14. Ultraviolet light is used to partially cure the adhesive. The position of the cover glass 68 is checked and, if the position is correct, the adhesive is exposed to more ultraviolet light to finish curing the adhesive. If the cover glass 68 is not in a correct position, it is readjusted while the adhesive remains partially cured.

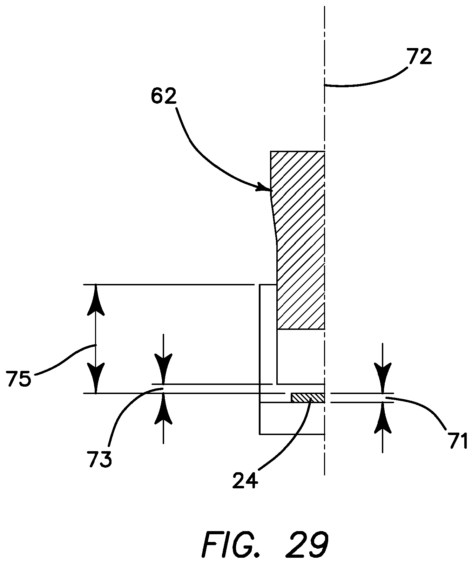

In accordance with various embodiments, the lens mount 66 includes a recessed pocket 82, also referred to as a recess, having a known depth. When the sensor 24 is placed in the pocket 82, it is located radially with respect to the shaft 14. Also, the depth of the pocket 82 within the lens mount 66 has a predetermined distance to the front of the lens mount 66. This distance effectively meets all magnification, working distance and depth-of-field requirements. Any tolerances found in the sensor or its electronics are accommodated by the varying distance between the lens mount 66 and sensor mount 30 as they sandwich the image sensor 24 and electronics. This simplifies assembly as the components fit with respect to each other in only one way with the sensor 24 always at a known distance from the end of the lens mount 66. Because of this, the adjustment of the lens housing 64 to optically tune the system is made easier. Otherwise, the need to tune the optics can vary from assembly to assembly because individual components have associated tolerances. When placed in relation to other components these tolerances can stack-up making tuning more difficult and leading to out-of-focus images. Tuning is also necessary as the distance from the backside of the lens to the image sensor affects magnification, working distance and the depth-of-field. The scope 10, in accordance with various embodiments, mitigates the tolerance stack-up issue. For example, as schematically illustrated in FIG. 29, the sensor 24 and lens 62 are shown relative to the longitudinal axis 72 and the tolerance 75 between the components is enhanced or otherwise maximized. In accordance with various embodiments, the thickness 71 of sensor 24 doesn't affect the tolerance stack-up as the top of the sensor remains in the same or fixed spot relative to the threaded side of the lens mount and the thickness 73 between the sensor and lens mount also doesn't affect the stack-up. In accordance with various embodiments, the sensor and/or lens mounts are configured to ensure the location of the sensor is within the depth of focus of the lens is maintained while not damaging the sensor. The sensor mount in various embodiments is configured to remove the unpredictability, difficulties and/or complexity in the coupling of the lens mount with the sensor mount with the sensor and connector there between.

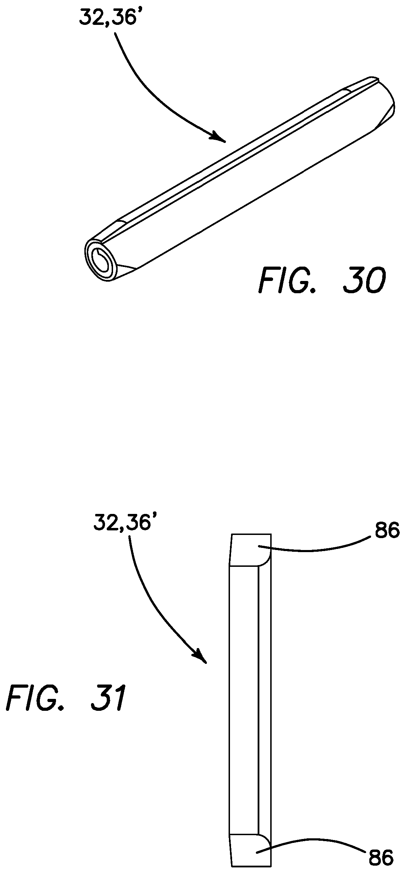

In FIGs. 30-31, there is shown a pin 32, 36'. In various embodiments, the pin 36' replaces the dowel 36. The pin 32, 36' is a spring pin that is rolled about itself and made of sufficiently flexible material to have a variable diameter. When compressed, the spring pin 32, 36', also called a roll pin, has a smaller diameter relative to when the spring pin 36' is relaxed. This variation in diameter assists insertion of the pin 32, 36' into pin holes 34 and 44, 72, respectively. When in a small diameter, compressed configuration, the pin 32, 36' can be easily inserted into the pin holes 34, 44 and when relaxed the relatively enlarged diameter assists in fixing the pin 32, 36' in place. The distal ends of the each pin 32, 36' include a slight taper 86.

In various embodiments, the scope has an elongated shaft that is connected to a handle at the proximal end. The shaft is long enough so that the distal end of the scope, which is the image acquisition end, is disposed inside the body cavity while the handle resides outside the patient. A scope includes an image sensor located behind a lens. The image sensor is connected to a controller printed circuit board assembly (PCBA) that includes a microcontroller configured to process data acquired by the image sensor, which is displayed on the monitor. The PCBA may be connected to the monitor or other device wirelessly or with a wire, cable or the like. Lights, such as LEDs or a fiber optic light source that transmits light through one or more fiber optic cables to the distal end of the scope, are included and connected to the scope and flexboard and/or controller PCBA to illuminate the surgical field. The lights are typically arranged in a circular fashion around the image acquisition end of the scope. One or more lenses are included in the optical assembly to focus the light reflected off the surgical site.

In order for the simulation surgical scopes in accordance with various embodiments of the present invention to be suitable or effective for training or simulation purposes, the scopes should meet one or more certain criteria. For example, the ergonomics of the scope should be similar to surgical-grade scopes. The size and shape of the handle, the length of the shaft as well as the weight of the scope should approximate a surgical-grade scope. Also, the simulation surgical scope should be capable of being manipulated by one hand. The scope should also be able to withstand accidental bumps and drops without breaking or losing functionality. Also, in order for users to learn and get comfortable with medical device trocars and the like, a simulation surgical scope should be sized and configured to be compatible with and fit inside medical device trocars that are used in surgery. Typically, there are two sizes of trocars, 10mm and 5mm, that are used as a port for passing a scope to the surgical site. Therefore, a simulation surgical scope should be at least be compatible with either a 10mm and/or 5mm trocar.

The simulation surgical scope in accordance with various embodiments does not need to be made to withstand repeated sterilization, cleaning and autoclave cycles. As such, the scope can be less expensive. While lighting on the scope can be required for certain procedure simulations, the light source at the tip of the scope in some instances can be supplemented by other light sources installed inside the cavity of the box trainer. Therefore, the lighting demands for a surgical simulation scope is reduced which may further lower manufacturing costs of the scope. Also, with respect to the optical performance, the simulation surgical scope in accordance with various embodiments may sacrifice some image quality and include some distortion at the edges of the image by using less expensive components and a simpler lens assembly design. Overall, the image quality should be sufficient enough to provide an appropriate amount of detail in order to distinguish the subtle differences in simulated anatomy. Since the requirements for image quality and lighting at the tip for a surgical simulation scope are not as stringent as that for a surgical-grade scope and there is no requirement for sterility, it is possible to keep the cost lower than that of surgical-grade scopes. A simulation surgical scope, in accordance with various embodiments, should have the balance of a quality image, working distance and depth-of-field, low cost, and/or robustness to last for a multitude of uses. These desired attributes can be connected and optimally designing a scope for one of these attributes may make meeting another attribute more difficult.

The surgical simulation scope in accordance with various embodiments is suitable for use with laparoscopic trainers to educate and train medical professionals and medical students. The surgical simulation scope in accordance with various embodiments provides significant improvements that may also be employed in surgical-grade scopes.

The above description is provided to enable any person skilled in the art to make and use the camera scopes and perform the methods described herein and sets forth the best modes contemplated by the inventors of carrying out their invention. Various modifications, however, may be apparent to those skilled in the art, which may be within the scope of the present invention, as defined by the following claims.

## Claims

1. A simulation surgical camera scope comprising:
a handle (12);
a cylindrical elongate shaft (14) having a proximal end coupled to the handle and a distal end comprising a cylindrical sensor mount (30) and a cylindrical lens mount (66), the cylindrical lens mount having threads disposed on an inner surface of the cylindrical lens mount;
a cylindrical lens (62) having threads disposed on an outer surface of the cylindrical lens (62) and arranged to mate with the threads of the cylindrical lens mount (66), the cylindrical lens mount (66) having a pocket (82), a central lumen (70) and at least one pin hole (72) positioned next to the pocket; and
an image sensor (24) disposed within the pocket (82) of the cylindrical lens mount (66) and between the cylindrical sensor mount (30) and the cylindrical lens mount, the cylindrical sensor mount (30) having a cavity (49) defined by a pair of raised surfaces (39), the image sensor (24) being disposed between the pair of raised surfaces (39) and between the pocket (82) of the cylindrical lens mount (66) and the cavity (49) defined by the pair of raised surfaces (39) of the cylindrical sensor mount (30), at least one of the pair of raised surface having at least one pin hole (34), the at least one pin hole (72) of the cylindrical lens mount (66) being aligned with the at least one pin hole (34) of the cylindrical sensor mount (30) and a pin (36) positioned within the at least one pin hole (72) of the cylindrical lens mount (66) and the at least one pin hole (34) of the cylindrical sensor mount, to align and hold both the lens mount and the sensor mount relative to each other.

2. The simulation surgical camera scope of claim 1 further comprising a flexible circuit board (26) extending from the distal end of the cylindrical elongate shaft (14) and into the handle (12).

3. The simulation surgical camera scope of claim 2 further comprising a controller printed circuit board assembly (PCBA) (20) positioned within the handle (12); and wherein flexible circuit board (26) is connected to the image sensor (24), extends along a length of the cylindrical elongate shaft (14), and is connected to the controller PCBA (20) within the handle (12).

4. The simulation surgical camera scope of claim 3, wherein the flexible circuit board (26) is configured to twist approximately 90 degrees within the cylindrical elongate shaft (14) to facilitate connection with the image sensor (24) and the controller PCBA (20).

5. The simulation surgical camera scope of claim 4, wherein the flexible circuit board (26) follows a S-shaped path starting from a distal end of the cylindrical lens mount (66), between the cylindrical lens mount (66) and the cylindrical sensor mount (30), past a distal end of the cylindrical sensor mount (30) and to the proximal end of the cylindrical elongate shaft (14).

6. The simulation surgical camera scope of claim 2 or 5, wherein the cylindrical sensor mount (30) has a proximal end, the proximal end of the cylindrical sensor mount (30) having an angled surface (48) interconnected with a flat portion (50), the angled surface (48) together with the flat portion (50) providing a landing and pathway for the flexible circuit board (26) extending proximally inside the cylindrical elongated shaft (14).

7. The simulation surgical camera scope of claim 6, wherein the distal end of the cylindrical sensor mount (30) includes a bevel (52) at an intersection of a distal end of the flat portion (50) of the cylindrical sensor mount (40), the bevel (52) providing a smooth bend for the flexible circuit board (26).

8. The simulation surgical camera scope of claim 7, wherein the cylindrical lens mount (66) comprises a sidewall comprising a flat portion (78) transitioning to a bevel (80), the flat portion (78) and the bevel (80) of the cylindrical lens mount (66) configured to provide room and a smooth transition for the flexible circuit board (26) as it bends approximately 90 degrees and extends in juxtaposition to the flat portion (78) of the cylindrical lens mount (66).

9. The simulation surgical camera scope of any one of claims 2 to 8, wherein the image sensor (24) and a portion of the flexible circuit board (26) are positioned on the cavity (49) defined by the pair of raised surfaces (39) of the cylindrical sensor mount (30) to prevent image sensor (24) from being crushed with the cylindrical sensor mount (30) and the cylindrical lens mount (66) being coupled together with the image sensor (24) and the portion of the flexible circuit board (26) therebetween.

10. The simulated surgical camera scope of any one of claims 2 to 9, wherein spacing between the cylindrical lens mount (66) and the cylindrical sensor mount (30) is greater than a thickness of the image sensor (24) and the flexible circuit board (26), the spacing being smaller than a depth of focus range of the cylindrical lens (62).

11. The simulation surgical camera scope of any one of claims 2 to 10, further comprising lights (28) including a plurality of LEDs connected to the flexible circuit board (26) and arranged in a circle around a central opening (58) and the cylindrical lens (62).

12. The simulation surgical camera scope of any one of claims 2 to 11, wherein the flexible circuit board (26) is a single planar monolithic component and further comprising a metallic layer configured to shield the flexible circuit board (26) from electrical noise.

13. The simulation surgical camera scope of any one of claims 2 to 12, wherein the cylindrical elongate shaft (14) includes a sidewall having a cylindrical shape defining a lumen surrounding the cylindrical sensor mount (30), the flexible circuit board (26), and the cylindrical lens mount (66); and wherein a first portion of the flexible circuit board (26) is positioned next to the cylindrical lens mount (66) and a second portion of the flexible circuit board (26) is positioned next the cylindrical sensor mount (30) and away from the first portion of the flexible circuit board (26).

14. The simulation surgical camera scope of claim 13, wherein a distance between the first portion of the flexible circuit board (26) to the second portion of the flexible circuit board (26) is greater than a length of the image sensor (24) and less than an inner diameter of the cylindrical elongate shaft (14); and wherein the first portion of the flexible circuit board (26) and the second portion of the flexible circuit board (26) are positioned parallel to each other.

15. The simulation surgical camera scope of any one of claims 1 to 14, wherein:
the cylindrical elongate shaft (14) has a central longitudinal axis;
the central lumen (70) of the cylindrical lens mount (66) has a central longitudinal axis offset from the central longitudinal axis of the cylindrical elongate shaft (14);
the raised surfaces (39) define a middle flat cavity (49) at the distal end of the cylindrical sensor mount (30) with a longitudinal axis of the cylindrical sensor mount (30) passing through the center of the middle flat cavity (49) being offset from both the central longitudinal axis of the cylindrical elongate shaft (14) and the central longitudinal axis of the central lumen of the cylindrical lens mount (66).

## Patentansprüche

1. Chirurgisches Simulationskameraskop, umfassend:
einen Griff (12);
einen zylindrischen länglichen Schaft (14) mit einem proximalen Ende, das an den Griff gekoppelt ist, und einem distalen Ende, das eine zylindrische Sensorfassung (30) und eine zylindrische Objektivfassung (66) umfasst, wobei die zylindrische Objektivfassung Gewindegänge aufweist, die auf einer inneren Oberfläche der zylindrischen Objektivfassung angeordnet sind;
ein zylindrisches Objektiv (62) mit Gewindegängen, die auf einer äußeren Oberfläche des zylindrischen Objektivs (62) angeordnet und dazu eingerichtet sind, mit den Gewindegängen der zylindrischen Objektivfassung (66) zusammenzupassen, wobei die zylindrische Objektivfassung (66) eine Tasche (82), ein zentrales Lumen (70) und mindestens ein Stiftloch (72) aufweist, das neben der Tasche positioniert ist; und
einen Bildsensor (24), der innerhalb der Tasche (82) der zylindrischen Objektivfassung (66) und zwischen der zylindrischen Sensorfassung (30) und der zylindrischen Objektivfassung angeordnet ist, wobei die zylindrische Sensorfassung (30) eine Aussparung (49) aufweist, die durch ein Paar von erhabenen Oberflächen (39) definiert ist, wobei der Bildsensor (24) zwischen dem Paar von erhabenen Oberflächen (39) und zwischen der Tasche (82) der zylindrischen Objektivfassung (66) und der Aussparung (49) angeordnet ist, die durch das Paar von erhabenen Oberflächen (39) der zylindrischen Sensorfassung (30) definiert ist, mindestens eine von dem Paar von erhabenen Oberflächen mindestens ein Stiftloch (34) aufweist, das mindestens eine Stiftloch (72) der zylindrischen Objektivfassung (66) auf das mindestens eine Stiftloch (34) der zylindrischen Sensorfassung (30) ausgerichtet wird und ein Stift (36) innerhalb des mindestens einen Stiftlochs (72) der zylindrischen Objektivfassung (66) und des mindestens einen Stiftlochs (34) der zylindrischen Sensorfassung positioniert wird, um sowohl die Objektivfassung als auch die Sensorfassung aufeinander auszurichten und zueinander zu halten.

2. Chirurgisches Simulationskameraskop nach Anspruch 1, weiterhin umfassend eine flexible Platine (26), die sich von dem distalen Ende des zylindrischen länglichen Schafts (14) und in den Griff (12) erstreckt.

3. Chirurgisches Simulationskameraskop nach Anspruch 2, weiterhin umfassend eine Controller-Leiterplattenbaugruppe (Controller-PCBA) (20), die innerhalb des Griffs (12) positioniert ist; und wobei eine flexible Platine (26) mit dem Bildsensor (24) verbunden ist, sich entlang einer Länge des zylindrischen länglichen Schafts (14) erstreckt und mit der Controller-PCBA (20) innerhalb des Griffs (12) verbunden ist.

4. Chirurgisches Simulationskameraskop nach Anspruch 3, wobei die flexible Platine (26) dazu konfiguriert ist, sich um ungefähr 90 Grad innerhalb des zylindrischen länglichen Schafts (14) zu verdrehen, um eine Verbindung mit dem Bildsensor (24) und der Controller-PCBA (20) zu erleichtern.

5. Chirurgisches Simulationskameraskop nach Anspruch 4, wobei die flexible Platine (26) einem S-förmigen Weg folgt, startend von einem distalen Ende der zylindrischen Objektivfassung (66), zwischen der zylindrischen Objektivfassung (66) und der zylindrischen Sensorfassung (30), an einem distalen Ende der zylindrischen Sensorfassung (30) vorbei und zu dem proximalen Ende des zylindrischen länglichen Schafts (14).

6. Chirurgisches Simulationskameraskop nach Anspruch 2 oder 5, wobei die zylindrische Sensorfassung (30) ein proximales Ende aufweist, wobei das proximale Ende der zylindrischen Sensorfassung (30) eine abgewinkelte Oberfläche (48) aufweist, die mit einem flachen Abschnitt (50) verknüpft ist, wobei die abgewinkelte Oberfläche (48) zusammen mit dem flachen Abschnitt (50) eine Auflage und einen Pfad für die flexible Platine (26) bereitstellt, die sich proximal im Inneren des zylindrischen länglichen Schafts (14) erstreckt.

7. Chirurgisches Simulationskameraskop nach Anspruch 6, wobei das distale Ende der zylindrischen Sensorfassung (30) eine Abschrägung (52) an einem Schnittpunkt eines distalen Endes des flachen Abschnitts (50) der zylindrischen Sensorfassung (40) einschließt, wobei die Abschrägung (52) eine glatte Biegung für die flexible Platine (26) bereitstellt.

8. Chirurgisches Simulationskameraskop nach Anspruch 7, wobei die zylindrische Objektivfassung (66) eine Seitenwand umfasst, umfassend einen flachen Abschnitt (78), der in eine Abschrägung (80) übergeht, wobei der flache Abschnitt (78) und die Abschrägung (80) der zylindrischen Objektivfassung (66) dazu konfiguriert sind, Platz und einen glatten Übergang für die flexible Platine (26) bereitzustellen, wenn sie sich um ungefähr 90 Grad biegt und sich gegenüber dem flachen Abschnitt (78) der zylindrischen Objektivfassung (66) erstreckt.

9. Chirurgisches Simulationskameraskop nach einem der Ansprüche 2 bis 8, wobei der Bildsensor (24) und ein Abschnitt der flexiblen Platine (26) an der Aussparung (49) positioniert sind, die durch das Paar von erhabenen Oberflächen (39) der zylindrischen Sensorfassung (30) definiert ist, um zu verhindern, dass ein Bildsensor (24) gequetscht wird, wenn die zylindrische Sensorfassung (30) und die zylindrische Objektivfassung (66) aneinander gekoppelt werden, wobei der Bildsensor (24) und der Abschnitt der flexiblen Platine (26) dazwischen sind.

10. Chirurgisches Simulationskameraskop nach einem der Ansprüche 2 bis 9, wobei ein Zwischenraum zwischen der zylindrischen Objektivfassung (66) und der zylindrischen Sensorfassung (30) größer als eine Dicke des Bildsensors (24) und der flexiblen Platine (26) ist, wobei der Zwischenraum kleiner als ein Tiefenschärfenbereich des zylindrischen Objektivs (62) ist.

11. Chirurgisches Simulationskameraskop nach einem der Ansprüche 2 bis 10, weiterhin umfassend Leuchten (28), einschließend eine Vielzahl von LED, die mit der flexiblen Platine (26) verbunden und in einem Kreis um eine zentrale Öffnung (58) und das zylindrische Objektiv (62) eingerichtet sind.

12. Chirurgisches Simulationskameraskop nach einem der Ansprüche 2 bis 11, wobei die flexible Platine (26) eine einzige planare monolithische Komponente ist und ferner eine metallische Schicht umfasst, die dazu konfiguriert ist, die flexible Platine (26) von elektrischem Rauschen abzuschirmen.

13. Chirurgisches Simulationskameraskop nach einem der Ansprüche 2 bis 12, wobei der zylindrische längliche Schaft (14) eine Seitenwand mit einer zylindrischen Form einschließt, die ein Lumen definiert, das die zylindrische Sensorfassung (30), die flexible Platine (26) und die zylindrische Objektivfassung (66) umgibt; und wobei ein erster Abschnitt der flexiblen Platine (26) neben der zylindrischen Objektivfassung (66) positioniert ist und ein zweiter Abschnitt der flexiblen Platine (26) neben der zylindrischen Sensorfassung (30) und von dem ersten Abschnitt der flexiblen Platine (26) weg positioniert ist.

14. Chirurgisches Simulationskameraskop nach Anspruch 13, wobei ein Abstand zwischen dem ersten Abschnitt der flexiblen Platine (26) und dem zweiten Abschnitt der flexiblen Platine (26) größer als eine Länge des Bildsensors (24) und kleiner als ein Innendurchmesser des zylindrischen länglichen Schafts (14) ist; und wobei der erste Abschnitt der flexiblen Platine (26) und der zweite Abschnitt der flexiblen Platine (26) parallel zueinander positioniert sind.

15. Chirurgisches Simulationskameraskop nach einem der Ansprüche 1 bis 14, wobei:
der zylindrische längliche Schaft (14) eine Mittellängsachse aufweist;
das zentrale Lumen (70) der zylindrischen Objektivfassung (66) eine Mittellängsachse aufweist, die von der Mittellängsachse des zylindrischen länglichen Schafts (14) versetzt ist;
die erhabenen Oberflächen (39) eine mittlere flache Aussparung (49) an dem distalen Ende der zylindrischen Sensorfassung (30) definieren, wobei eine Längsachse der zylindrischen Sensorfassung (30) durch die Mitte der mittleren flachen Aussparung (49) verläuft, die von sowohl der Mittellängsachse des zylindrischen länglichen Schafts (14) als auch der Mittellängsachse des zentralen Lumens der zylindrischen Objektivfassung (66) versetzt ist.

## Revendications

1. Viseur de caméra chirurgicale de simulation comprenant :
une poignée (12) ;
une tige allongée cylindrique (14) ayant une extrémité proximale couplée à la poignée et une extrémité distale comprenant un support de capteur cylindrique (30) et un support de lentille cylindrique (66), le support de lentille cylindrique ayant des fils disposés sur une surface interne du support de lentille cylindrique ;
une lentille cylindrique (62) ayant des fils disposés sur une surface externe de la lentille cylindrique (62) et agencés pour s'accoupler avec les fils du support de lentille cylindrique (66), le support de lentille cylindrique (66) ayant une poche (82), une lumière centrale (70) et au moins un trou de broche (72) positionné à côté de la poche ; et
un capteur d'image (24) disposé au sein de la poche (82) du support de lentille cylindrique (66) et entre le support de capteur cylindrique (30) et le support de lentille cylindrique, le support de capteur cylindrique (30) ayant une cavité (49) définie par une paire de surfaces surélevées (39), le capteur d'images (24) étant disposé entre la paire de surfaces surélevées (39) et entre la poche (82) du support de lentille cylindrique (66) et la cavité (49) définie par la paire de surfaces surélevées (39) du support de capteur cylindrique (30), au moins l'une de la paire de surfaces surélevées ayant au moins un trou de broche (34), l'au moins un trou de broche (72) du support de lentille cylindrique (66) étant aligné avec l'au moins un trou de broche (34) du support de capteur cylindrique (30) et une broche (36) positionnée au sein de l'au moins un trou de broche (72) du support de lentille cylindrique (66) et de l'au moins un trou de broche (34) du support de capteur cylindrique, pour s'aligner et retenir les deux support de lentille et support de capteur l'un avec l'autre.

2. Viseur de caméra chirurgicale de simulation selon la revendication 1 comprenant en outre une carte de circuit flexible (26) s'étendant à partir de l'extrémité distale de la tige allongée allongé cylindrique (14) et jusque dans la poignée (12).

3. Viseur de caméra chirurgicale de simulation selon la revendication 2 comprenant en outre un ensemble de carte de circuit imprimé (PCBA) de dispositif de commande (20) positionné au sein de la poignée (12) ; et dans lequel la carte de circuit flexible (26) est connectée au capteur d'image (24), s'étend le long d'une longueur de la tige allongée allongé cylindrique (14), et est connectée au PCBA de dispositif de commande (20) au sein de la poignée (12).

4. Viseur de caméra chirurgicale de simulation selon la revendication 3, dans lequel la carte de circuit flexible (26) est configurée pour se tordre d'approximativement 90 degrés au sein de l'arbre allongé cylindrique (14) pour faciliter la connexion avec le capteur d'image (24) et le PCBA de dispositif de commande (20).

5. Viseur de caméra chirurgicale de simulation selon la revendication 4, dans lequel la carte de circuit flexible (26) a une forme de S partant d'une extrémité distale du support de lentille cylindrique (66), entre le support de lentille cylindrique (66) et le support de capteur cylindrique (30), devant une extrémité distale du support de capteur cylindrique (30) et vers l'extrémité proximale de l'arbre allongé cylindrique (14).

6. Viseur de caméra chirurgicale de simulation selon l'une quelconque des revendications 2 ou 5, dans lequel le support de capteur cylindrique (30) a une extrémité proximale, l'extrémité proximale du support de capteur cylindrique (30) ayant une surface inclinée (48) interconnectée avec une partie plate (50), la surface inclinée (48) ainsi que la partie plate (50) fournissant un élément d'accueil et une voie de passage pour la carte de circuit flexible (26) s'étendant de manière proximale à l'intérieur de l'arbre allongé cylindrique (14).

7. Viseur de caméra chirurgicale de simulation selon la revendication 6, dans lequel l'extrémité distale du support de capteur cylindrique (30) inclut un biseau (52) au niveau d'une intersection d'une extrémité distale de la partie plate (50) du support de capteur cylindrique (40), le biseau (52) fournissant une courbe lisse pour la carte de circuit flexible (26).

8. Viseur de caméra chirurgicale de simulation selon la revendication 7, dans lequel le support de lentille cylindrique (66) comprend une paroi latérale comprenant une partie plate (78) effectuant une transition vers un biseau (80), la partie plate (78) et le biseau (80) du support de lentille cylindrique (66) configurés pour fournir de la place et une transition lisse pour la carte de circuit flexible (26) alors qu'elle s'incurve d'approximativement 90 degrés et s'étend en juxtaposition vers la partie plate (78) du support de lentille cylindrique (66).

9. Viseur de caméra chirurgicale de simulation selon l'une quelconque des revendications 2 à 8, dans lequel le capteur d'image (24) et une partie de la carte de circuit flexible (26) sont positionnés sur la cavité (49) définie par la paire de surfaces surélevées (39) du support de capteur cylindrique (30) pour empêcher que le capteur d'image (24) soit écrasé avec le support de capteur cylindrique (30) et le support de lentille cylindrique (66) étant couplée avec le capteur d'image (24) et la partie de la carte de circuit flexible (26) entre ceux-là.

10. Viseur de caméra chirurgicale de simulation selon l'une quelconque des revendications 2 à 9, dans lequel l'espacement entre le support de lentille cylindrique (66) et le support de capteur cylindrique (30) est supérieur à une épaisseur du capteur d'image (24) et de la carte de circuit flexible (26), l'espacement étant inférieur à une plage de profondeur de mise au point de la lentille cylindrique (62).

11. Viseur de caméra chirurgicale de simulation selon l'une quelconque des revendications 2 à 10, comprenant en outre des lumières (28) incluant une pluralité de DEL connectées à la carte de circuit flexible (26) et agencées en cercle autour d'une ouverture centrale (58) et de la lentille cylindrique (62).

12. Viseur de caméra chirurgicale de simulation selon l'une quelconque des revendications 2 à 11, dans lequel la carte de circuit flexible (26) est un composant monolithique plan unique et comprenant en outre une couche métallique configurée pour protéger la carte de circuit flexible (26) contre le bruit électrique.

13. Viseur de caméra chirurgicale de simulation selon l'une quelconque des revendications 2 à 12, dans lequel la tige allongée (14) inclut une paroi latérale ayant une forme cylindrique définissant une lumière entourant le support de capteur cylindrique (30), la carte de circuit flexible (26), et le support de lentille cylindrique (66) ; et dans lequel une première partie de la carte de circuit flexible (26) est positionnée à côté du support de lentille cylindrique (66) et une deuxième partie de la carte de circuit flexible (26) est positionnée à côté du support de capteur cylindrique (30) et à distance de la première partie de la carte de circuit flexible (26).

14. Viseur de caméra chirurgicale de simulation selon la revendication 13, dans lequel une distance entre la première partie de la carte de circuit flexible (26) à la deuxième partie de la carte de circuit flexible (26) est supérieure à une longueur du capteur d'image (24) et inférieure à un diamètre interne de la tige allongée cylindrique (14) ; et dans lequel la première partie de la carte de circuit flexible (26) et la deuxième partie de la carte de circuit flexible (26) sont positionnées parallèles l'une à l'autre.

15. Viseur de caméra chirurgicale de simulation selon l'une quelconque des revendications 1 à 14, dans lequel :
la tige allongée cylindrique (14) a un axe longitudinal central ;
la lumière centrale (70) du support de lentille cylindrique (66) a un axe longitudinal décalé par rapport à l'axe longitudinal central de l'arbre allongé cylindrique (14) ;
les surfaces surélevées (39) définissent une cavité plate moyenne (49) au niveau de l'extrémité distale du support de capteur cylindrique (30) avec un axe longitudinal du support de capteur cylindrique (30) passant à travers le centre de la cavité plate moyenne (49) étant décalé par rapport à la fois à l'axe longitudinal central de la tige allongée cylindrique (14) et de l'axe longitudinal central de la lumière centrale du support de lentille cylindrique (66).
